# EUROPEAN PATENT APPLICATION

(11) **EP 3 330 711 A1**
(43) Date of publication of application: **06.06.2018**
(21) Application number: 17208382.6
(22) Date of filing: 26.03.2012
(51) Int. Cl.: G01N 33/68, C12Q 1/6883

(54) **METHODS OF DIAGNOSING AND TREATING INTESTINAL GRANULOMAS AND LOW BONE DENSITY IN INFLAMMATORY BOWEL DISEASE**

(30) Priority: 25.03.2011 US 201161467779 P; 25.03.2011 US 201161467881 P
(62) Divisional of application: 12765854.0
(71) Applicant: Cedars-Sinai Medical Center, Los Angeles, CA 90048 (US)
(72) Inventor: McGOVERN, Dermot P., Los Angeles, CA 90049 (US); DUBINSKY, Marla C., Los Angeles, CA 90048 (US); TAYLOR, Kent D., Ventura, CA 93004 (US); TARGAN, Stephan R., Santa Monica, CA 90402 (US); ROTTER, Jerome I., Los Angeles, CA 90064 (US)
(74) Representative: HGF Limited

(57) **Abstract**

The present invention relates to methods of diagnosing inflammatory bowel disease (IBD) in an individual by determining the presence of at least one risk genetic variant and/or at least one risk serological marker. In one embodiment, the presence of at least one risk genetic variant is indicative of granuloma. In another embodiment, the presence of at least one risk genetic variant is indicative of low bone density (LBD).

## Description

### FIELD OF INVENTION

The invention relates to the field of genetics and medicine. More specifically, the invention relates to methods of diagnosing and treating inflammatory bowel disease including ulcerative colitis and Crohn's disease.

### BACKGROUND

All publications herein are incorporated by reference to the same extent as if each individual publication or patent application was specifically and individually indicated to be incorporated by reference. The following description includes information that may be useful in understanding the present invention. It is not an admission that any of the information provided herein is prior art or relevant to the presently claimed invention, or that any publication specifically or implicitly referenced is prior art.

Crohn's disease (CD) and ulcerative colitis (UC), the two common forms of idiopathic inflammatory bowel disease (IBD), are chronic, relapsing inflammatory disorders of the gastrointestinal tract. Each has a peak age of onset in the second to fourth decades of life and prevalences in European ancestry populations that average approximately 100-150 per 100,000 (D.K. Podolsky, N Engl J Med 347, 417 (2002); E.V. Loftus, Jr., Gastroenterology 126, 1504 (2004)). Although the precise etiology of IBD remains to be elucidated, a widely accepted hypothesis is that ubiquitous, commensal intestinal bacteria trigger an inappropriate, overactive, and ongoing mucosal immune response that mediates intestinal tissue damage in genetically susceptible individuals (D.K. Podolsky, N Engl J Med 347, 417 (2002)). Genetic factors play an important role in IBD pathogenesis, as evidenced by the increased rates of IBD in Ashkenazi Jews, familial aggregation of IBD, and increased concordance for IBD in monozygotic compared to dizygotic twin pairs (S. Vermeire, P. Rutgeerts, Genes Immun 6, 637 (2005)). Moreover, genetic analyses have linked IBD to specific genetic variants, especially CARD15 variants on chromosome 16q12 and the IBD5 haplotype (spanning the organic cation transporters, SLC22A4 and SLC22A5, and other genes) on chromosome 5q31 (S. Vermeire, P. Rutgeerts, Genes Immun 6, 637 (2005); J.P. Hugot et al., Nature 411, 599 (2001); Y. Ogura et al., Nature 411, 603 (2001); J.D. Rioux et al., Nat Genet 29, 223 (2001); V.D. Peltekova et al., Nat Genet 36, 471 (2004)). CD and UC are thought to be related disorders that share some genetic susceptibility loci but differ at others.

### SUMMARY OF THE INVENTION

Various embodiments include a method of diagnosing susceptibility to granuloma in an individual with Crohn's diseaes, comprising obtaining a sample from the individual, assaying the sample to determine the presence or absence of at least one risk genetic variant, assaying the sample to determine the presence or absence of at least one risk serological marker, and diagnosing susceptibility to granuloma in the individual if the at least one risk genetic variant is present, or if the at least one risk serological marker is present, or if the at least one risk genetic variant is present and the at least one risk serological marker is present. In another embodiment, the at least one risk genetic variant is at the genetic locus of TGFb3, FTO, NPAS2, MUC1, IL10, LRAP, LRRK2, TNFSF15, or cytochrome P-450 cluster, or a combination thereof. In another embodiment, the at least one risk serological marker is selected from the group consisting of anti-Cbirl, ANCA, ASCA, anti-OmpC, and anti-I2. In another embodiment, the ASCA is present in high titre. In another embodiment, the at least one risk genetic variant includes SEQ. ID. NO.: 1, SEQ. ID. NO.: 2, SEQ. ID. NO.: 3, SEQ. ID. NO.: 4, SEQ. ID. NO. : 5, and/or SEQ. ID. NO.: 6. In another embodiment, the at least one risk genetic variant includes SEQ. ID. NO.: 7, SEQ. ID. NO.: 8, SEQ. ID. NO.: 9, SEQ. ID. NO.: 10, SEQ. ID. NO. : 11, SEQ. ID. NO.: 12, and/or SEQ. ID. NO.: 13. In another embodiment, the Crohn's disease is associated with a small bowel disease phenotype, an aggressive complicating phenotype, an internal penetrating disease phenotype, a stricturing disease phenotype, or a fibrostenosing disease phenotype, or a combination thereof. In another embodiment, the first and/or second sample comprises a nucleic acid from the individual.

Other embodiments include a method of diagnosing granuloma in an individual with Crohn's disease, comprising obtaining a sample from the individual, and assaying the sample to determine the presence or absence of at least one risk genetic variant, assaying the sample to determine the presence or absence of at least one risk serological marker, and diagnosing granuloma in the individual if the at least one risk genetic variant is present, or if the at least one risk serological marker is present, or if the at least one risk genetic variant is present and the at least one risk serological marker is present. In another embodiment, the at least one risk genetic variant is at the genetic locus of TGFb3, FTO, NPAS2, MUC1, IL10, LRAP, LRRK2, TNFSF15, or cytochrome P-450 cluster, or a combination thereof. In another embodiment, the at least one risk serological marker is selected from the group consisting of anti-Cbirl, ANCA, ASCA, anti-OmpC, and anti-I2. In another embodiment, the ASCA is present in high titre. In another embodiment, the at least one risk genetic variant includes SEQ. ID. NO.: 1, SEQ. ID. NO.: 2, SEQ. ID. NO.: 3, SEQ. ID. NO.: 4, SEQ. ID. NO. : 5, and/or SEQ. ID. NO.: 6. In another embodiment, the at least one risk genetic variant includes SEQ. ID. NO.: 7, SEQ. ID. NO.: 8, SEQ. ID. NO.: 9, SEQ. ID. NO.: 10, SEQ. ID. NO. : 11, SEQ. ID. NO.: 12, and/or SEQ. ID. NO.: 13. In another embodiment, the Crohn's disease is associated with a small bowel disease phenotype, an aggressive complicating phenotype, an internal penetrating disease phenotype, a stricturing disease phenotype, or a fibrostenosing disease phenotype, or a combination thereof.

Various embodiments include a method of diagnosing susceptibility to low bone density (LBD) in an individual with an inflammatory bowel disease (IBD), comprising obtaining a sample from the individual, assaying the sample to determine the presence or absence of at least one risk genetic variant, assaying the sample to determine the presence or absence of at least one risk serological marker, and diagnosing susceptibility to LBD in the individual if the at least one risk genetic variant is present, or if the at least one risk serological marker is present, or if the at least one risk genetic variant is present and the at least one risk serological marker is present. In another embodiment, the LBD is osteoporosis or osteopenia. In another embodiment, the at least one risk genetic variant is at the genetic locus of HLA, laminin, plexin, or NLR family, or a combination thereof. In another embodiment, the at least one risk genetic variant is SEQ. ID. NO.: 14 and/or SEQ. ID. NO.: 15. In another embodiment, the at least one risk serological marker is selected from the group consisting of anti-Cbirl, ASCA, and anti-I2. In another embodiment, the IBD is a perianal disease.

Other embodiments include a method of treating low bone density (LBD) in an individual with an inflammatory bowel disease (IBD), comprising obtaining a sample from the individual, assaying the sample to determine the presence or absence of at least one risk genetic variant, assaying the sample to determine the presence or absence of at least one risk serological marker, and treating LBD in the individual if the at least one risk genetic variant is present, or if the at least one risk serological marker is present, or if the at least one risk genetic variant is present and the at least one risk serological marker is present. In another embodiment, the at least one risk genetic variant is SEQ. ID. NO.: 14 and/or SEQ. ID. NO.: 15. In another embodiment, the at least one risk serological marker is selected from the group consisting of anti-Cbirl, ASCA, and anti-I2.

Other features and advantages of the invention will become apparent from the following detailed description, taken in conjunction with the accompanying drawings, which illustrate, by way of example, various embodiments of the invention.

### BRIEF DESCRIPTION OF THE FIGURES

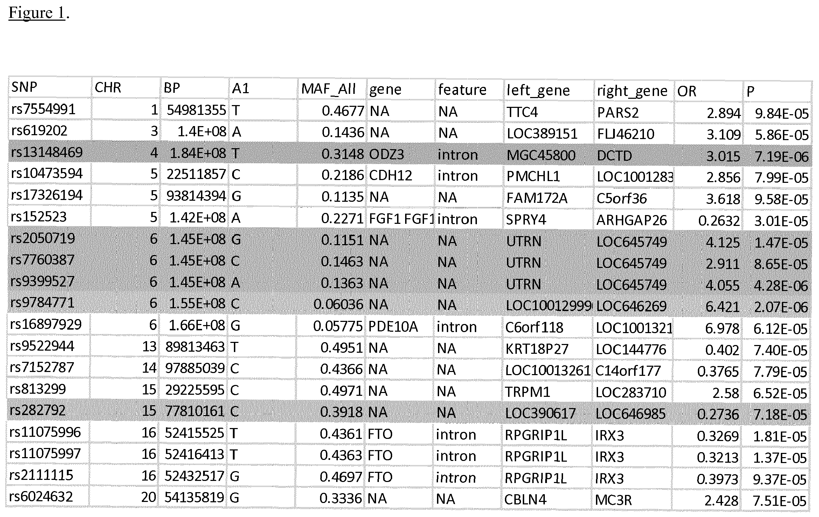

Figure 1 depicts top hits of various listed SNPs and corresponding alleles as associated with granuloma from performed GWAS.

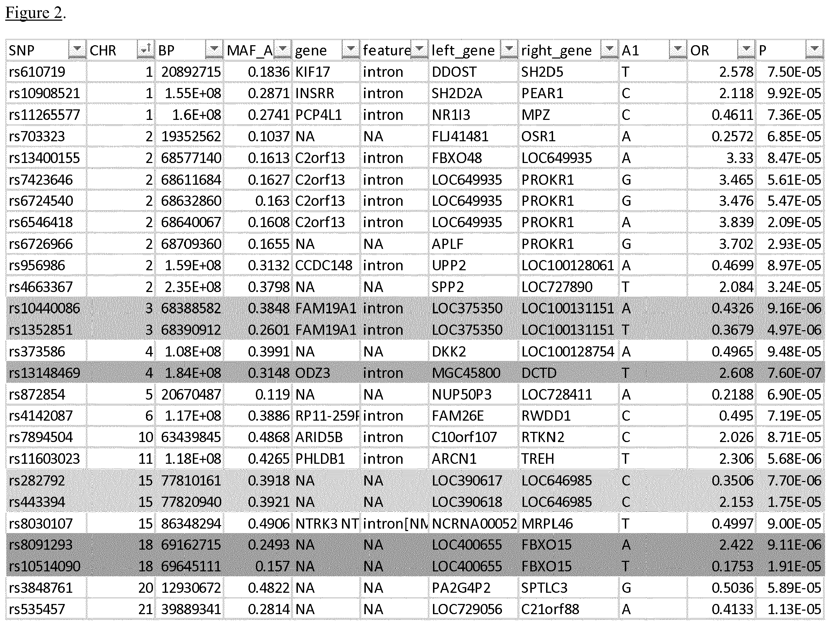

Figure 2 depicts top hits of various listed SNPs and corresponding alleles as associated with granuloma from performed GWAS.

### DESCRIPTION OF THE INVENTION

All references cited herein are incorporated by reference in their entirety as though fully set forth. Unless defined otherwise, technical and scientific terms used herein have the same meaning as commonly understood by one of ordinary skill in the art to which this invention belongs. Singleton et al., Dictionary of Microbiology and Molecular Biology 3rd ed., J. Wiley & Sons (New York, NY 2001); March, Advanced Organic Chemistry Reactions, Mechanisms and Structure 5th ed., J. Wiley & Sons (New York, NY 2001); and Sambrook and Russel, Molecular Cloning: A Laboratory Manual 3rd ed., Cold Spring Harbor Laboratory Press (Cold Spring Harbor, NY 2001), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described.

"IBD" as used herein is an abbreviation of inflammatory bowel disease.

"CD" as used herein is an abbreviation of Crohn's Disease.

"SNP" as used herein is an abbreviation of single nucleotide polymorphism.

As readily understood by one of skill in the art, any number of sequences may also be used to obtain the various SNPs or genetic variants referenced herein, and the variants are not limited to the specific sequences or accession numbers provided herein. Examples of SNPs rs13148469, rs2050719, rs7760387, rs9399527, rs9784771, rs282792 are provided herein as SEQ. ID. NO.: 1, SEQ. ID. NO.: 2, SEQ. ID. NO.: 3, SEQ. ID. NO.: 4, SEQ. ID. NO. : 5, and SEQ. ID. NO.: 6, respectively. Similarly, examples of SNPs rs10440086, rs1352851, rs13148469, rs282792, rs443394, rs8091293, and rs10514090 are provided herein as SEQ. ID. NO.: 7, SEQ. ID. NO.: 8, SEQ. ID. NO.: 9, SEQ. ID. NO.: 10, SEQ. ID. NO. : 11, SEQ. ID. NO.: 12, and SEQ. ID. NO.: 13, respectively.

In accordance with low bone density studies referenced herein, examples of SNPs rs11576349 and rs4954555 are provided herein as SEQ. ID. NO.: 14 and SEQ. ID. NO.: 15, respectively.

As used herein, the term "biological sample" means any biological material from which nucleic acid molecules can be prepared. As non-limiting examples, the term material encompasses whole blood, plasma, saliva, cheek swab, or other bodily fluid or tissue that contains nucleic acid.

As disclosed herein, the inventors identified clinical, serologic and genetic factors associated with granuloma formation in Crohn's disease (CD). 371 patients with CD who underwent disease-related surgical resection by a single surgeon were included in the study. Surgical samples were examined specifically for the presence or not of granulomas. Patients' demographic and clinical characteristics were collected by chart review, and samples drawn for IBD related serology (ASCA, anti-I2, anti-OmpC, CBirl and ANCA) and genetic analyses. Genome-wide analyses were performed using Illumina technology. Standard statistical tests for association were used and genetic association was assessed both at the genome-wide level and against known IBD and Leprosy susceptibility loci.

As further disclosed herein, 34.7% of CD surgical samples were found to contain granulomas. Granulomas were not associated with CD disease behavior. High ASCA titer was associated with the presence of granulomas (p=0.02). Patients with granulomas were younger at time of surgery (29.9 vs. 37.6 years, p=5x10-7) and far less likely to have ever smoked (12 vs. 32%, p=7x10-5). 14 Single Nucleotide Polymorphisms (SNPs) were associated with granulomas at a level of nominal association at a genome-wide level (p< 0.00005). These include a SNP adjacent to TGFb3, which has been implicated in the pathogenesis of stricturing Crohn's disease, and FTO, which is regulated by oral intake and is associated with raised body mass index. The strongest association was with NPAS2 (p =1x10-6), a core circadian gene that has been shown to modulate transcription of CX3CL1, a chemokine involved in CD pathogenesis. Amongst known IBD-associated loci, 7 were associated with granuloma formation (p<0.05), including: MUC1 (KL-6), also associated with granuloma-forming hypersensitivity pneumonitis; IL10, with known immunoregulatory function in the gut; and LRAP, associated with antigen presentation and LRRK2 a leucine-rich rpeat kinase gene. One TNFSF15 SNP showed a trend towards association with the presence of granulomas (P = 0.066), of particular interest given a recent report that TNFSF15 is associated with Leprosy, another granulomatous condition. Of the known Leprosy loci (in addition to LRRK2 and TNFSF15), the inventors identified association with granulomatous CD and SNPs across the cytochrome P-450 cluster. Thus, the inventors have demonstrated putative genetic and demographic associations with the presence of granulomas in CD including a number of genes associated with Leprosy suggesting unique pathways in the pathogenesis of this subset of CD.

In one embodiment, the present invention provides a method of diagnosing susceptibility to a subgroup of Crohn's disease in an individual, by obtaining a sample from the individual, and assaying the sample to determine the presence or absence of one or more genetic risk variants and/or risk serological markers, wherein the presence of one or more genetic risk variants and/or risk serological markers is indicative of susceptibility to the subgroup of Crohn's disease. In another embodiment, the subgroup of Crohn's disease is characterized by granuloma manifestations. In another embodiment, the one or more genetic risk variants are at the genetic loci of TGFb3, FTO, NPAS2, MUC1, IL10, LRAP, LRRK2, TNFSF15, and/or cytochrome p450 cluster. In another embodiment, the one or more risk serological markers include a high expression level of ASCA relative to a healthy subject.

In one embodiment, the present invention provides a method of treating Crohn's disease in an individual by determining the presence of one or more genetic risk variants associated with granulomas, and treating the individual.
As disclosed herein, the inventors identified 333 IBD subjects with bone density studies who had previously had genome wide association studies and IBD related serologies performed. Data on age, gender, ethnicity, disease distribution, surgeries, and smoking history were obtained from chart reviews. Osteoporosis, osteopenia, and normal bone mineral density (NBD) were defined by the WHO criteria based on DEXA scans. Standard tests for association between clinical characteristics, genetic markers and serologies were used. IBD related serology (ASCA, OmpC, 12, CBir-1, and ANCA) were obtained by ELISA and summarized into quartiles. Genetic data were generated using Illumina technology.

As further disclosed herein, of the 333 IBD study subjects, the inventors identified 252 cases of low bone density (LBD) and 81 cases of NBD. Disease location was not associated with LBD overall; however, perianal disease was associated with osteoporosis (P=0.021). Small bowel disease requiring surgery was associated with LBD (P=0.022), osteopenia (P=0.041) and osteoporosis (P=0.05). Smoking was not associated with bone density. Mean and median Anti-I2 titers were associated with LBD (P=0.023) and osteoporosis (P=0.006). On quartile analysis, anti-CBirl titers were associated with LBD (P=0.036) and osteoporosis (P=0.0006); further, ASCA was associated with osteoporosis (P=0.03). 38 genetic loci achieved nominal level of genome wide significance (P<5x10⁻⁵) including multiple single nucleotide polymorphisms (SNPs) at the HLA (P=1.37x10⁻⁷) as well as genes involved in cell adhesion (laminin, P=4.41x10⁻⁵) and innate immunity (plexin, P=9.02x10⁻⁷ ; NLR family, P=7.39x10⁻⁶). Stepwise linear regression was performed and all but 2 SNPs (rs 11576349 and rs4954555) fell out of the model. These two SNPs were independently associated with LBD (2.41 x10⁻⁵ and 1.07 x 10⁻⁵) and together this 2 SNP model was highly associated with LBD (p-value linear regression 1.8 x 10⁻⁹) and explained 12.6 of the variance. Perianal disease is associated with osteoporosis; further, small bowel disease requiring surgery increases the risk for LBD. Anti-I2, anti-CBirl, and ASCA are associated with increased risk for LBD and/or osteoporosis. Genes including HLA, laminin and plexin are associated with LBD. Thus, patients with these risk factors may benefit from more aggressive screening and treatment for osteoporosis.

In one embodiment, the present invention provides a method of diagnosing susceptibility to a condition characterized by low bone density in an individual by obtaining a sample from the individual, assaying the sample to determine the presence or absence of one or more risk factors and/or risk serological markers, where the presence of one or more genetic risk factors and/or risk serological markers is indicative of susceptibility to a condition characterized by low bone density in the individual. In another embodiment, the individual is diagnosed with inflammatory bowel disease (IBD). In another embodiment, the one or more risk factors include genetic risk variants at the genetic loci of HLA, laminin, and/or plexin. In another embodiment, the presence of perianal disease is associated with an increased risk of osteoporosis. In another embodiment, the presence of small bowel disease requiring surgery is associated with an increased risk of susceptibility to LBD, osteopenia, and/or osteoporosis. In another embodiment, the one or more risk serological markers include 12, Cbir1, and/or ASCA.

In one embodiment, the present invention provides a method of treating a condition characterized by low bone density in an individual, by determining the presence of one or more risk factors and/or serological markers, and treating the individual.

A variety of methods can be used to determine the presence or absence of a variant allele or haplotype. As an example, enzymatic amplification of nucleic acid from an individual may be used to obtain nucleic acid for subsequent analysis. The presence or absence of a variant allele or haplotype may also be determined directly from the individual's nucleic acid without enzymatic amplification.

Analysis of the nucleic acid from an individual, whether amplified or not, may be performed using any of various techniques. Useful techniques include, without limitation, polymerase chain reaction based analysis, sequence analysis and electrophoretic analysis. As used herein, the term "nucleic acid" means a polynucleotide such as a single or double-stranded DNA or RNA molecule including, for example, genomic DNA, cDNA and mRNA. The term nucleic acid encompasses nucleic acid molecules of both natural and synthetic origin as well as molecules of linear, circular or branched configuration representing either the sense or antisense strand, or both, of a native nucleic acid molecule.

The presence or absence of a variant allele or haplotype may involve amplification of an individual's nucleic acid by the polymerase chain reaction. Use of the polymerase chain reaction for the amplification of nucleic acids is well known in the art (see, for example, Mullis et al. (Eds.), The Polymerase Chain Reaction, Birkhauser, Boston, (1994)).

A TaqmanB allelic discrimination assay available from Applied Biosystems may be useful for determining the presence or absence of a variant allele. In a TaqmanB allelic discrimination assay, a specific, fluorescent, dye-labeled probe for each allele is constructed. The probes contain different fluorescent reporter dyes such as FAM and VICTM to differentiate the amplification of each allele. In addition, each probe has a quencher dye at one end which quenches fluorescence by fluorescence resonant energy transfer (FRET). During PCR, each probe anneals specifically to complementary sequences in the nucleic acid from the individual. The 5' nuclease activity of Taq polymerase is used to cleave only probe that hybridize to the allele. Cleavage separates the reporter dye from the quencher dye, resulting in increased fluorescence by the reporter dye. Thus, the fluorescence signal generated by PCR amplification indicates which alleles are present in the sample. Mismatches between a probe and allele reduce the efficiency of both probe hybridization and cleavage by Taq polymerase, resulting in little to no fluorescent signal. Improved specificity in allelic discrimination assays can be achieved by conjugating a DNA minor grove binder (MGB) group to a DNA probe as described, for example, in Kutyavin et al., "3'-minor groove binder-DNA probes increase sequence specificity at PCR extension temperature, "Nucleic Acids Research 28:655-661 (2000)). Minor grove binders include, but are not limited to, compounds such as dihydrocyclopyrroloindole tripeptide (DPI,).

Sequence analysis also may also be useful for determining the presence or absence of a variant allele or haplotype.

Restriction fragment length polymorphism (RFLP) analysis may also be useful for determining the presence or absence of a particular allele (Jarcho et al. in Dracopoli et al., Current Protocols in Human Genetics pages 2.7.1-2.7.5, John Wiley & Sons, New York; Innis et al.,(Ed.), PCR Protocols, San Diego: Academic Press, Inc. (1990)). As used herein, restriction fragment length polymorphism analysis is any method for distinguishing genetic polymorphisms using a restriction enzyme, which is an endonuclease that catalyzes the degradation of nucleic acid and recognizes a specific base sequence, generally a palindrome or inverted repeat. One skilled in the art understands that the use of RFLP analysis depends upon an enzyme that can differentiate two alleles at a polymorphic site.

Allele-specific oligonucleotide hybridization may also be used to detect a disease-predisposing allele. Allele-specific oligonucleotide hybridization is based on the use of a labeled oligonucleotide probe having a sequence perfectly complementary, for example, to the sequence encompassing a disease-predisposing allele. Under appropriate conditions, the allele-specific probe hybridizes to a nucleic acid containing the disease-predisposing allele but does not hybridize to the one or more other alleles, which have one or more nucleotide mismatches as compared to the probe. If desired, a second allele-specific oligonucleotide probe that matches an alternate allele also can be used. Similarly, the technique of allele-specific oligonucleotide amplification can be used to selectively amplify, for example, a disease-predisposing allele by using an allele-specific oligonucleotide primer that is perfectly complementary to the nucleotide sequence of the disease-predisposing allele but which has one or more mismatches as compared to other alleles (Mullis et al., supra, (1994)). One skilled in the art understands that the one or more nucleotide mismatches that distinguish between the disease-predisposing allele and one or more other alleles are preferably located in the center of an allele-specific oligonucleotide primer to be used in allele-specific oligonucleotide hybridization. In contrast, an allele-specific oligonucleotide primer to be used in PCR amplification preferably contains the one or more nucleotide mismatches that distinguish between the disease-associated and other alleles at the 3' end of the primer.

A heteroduplex mobility assay (HMA) is another well known assay that may be used to detect a SNP or a haplotype. HMA is useful for detecting the presence of a polymorphic sequence since a DNA duplex carrying a mismatch has reduced mobility in a polyacrylamide gel compared to the mobility of a perfectly base-paired duplex (Delwart et al., Science 262:1257-1261 (1993); White et al., Genomics 12:301-306 (1992)).

The technique of single strand conformational, polymorphism (SSCP) also may be used to detect the presence or absence of a SNP and/or a haplotype (see Hayashi, K., Methods Applic. 1:34-38 (1991)). This technique can be used to detect mutations based on differences in the secondary structure of single-strand DNA that produce an altered electrophoretic mobility upon non-denaturing gel electrophoresis. Polymorphic fragments are detected by comparison of the electrophoretic pattern of the test fragment to corresponding standard fragments containing known alleles.

Denaturing gradient gel electrophoresis (DGGE) also may be used to detect a SNP and/or a haplotype. In DGGE, double-stranded DNA is electrophoresed in a gel containing an increasing concentration of denaturant; double-stranded fragments made up of mismatched alleles have segments that melt more rapidly, causing such fragments to migrate differently as compared to perfectly complementary sequences (Sheffield et al., "Identifying DNA Polymorphisms by Denaturing Gradient Gel Electrophoresis" in Innis et al., supra, 1990).

Other molecular methods useful for determining the presence or absence of a SNP and/or a haplotype are known in the art and useful in the methods of the invention. Other well-known approaches for determining the presence or absence of a SNP and/or a haplotype include automated sequencing and RNAase mismatch techniques (Winter et al., Proc. Natl. Acad. Sci. 82:7575-7579 (1985)). Furthermore, one skilled in the art understands that, where the presence or absence of multiple alleles or haplotype(s) is to be determined, individual alleles can be detected by any combination of molecular methods. See, in general, Birren et al. (Eds.) Genome Analysis: A Laboratory Manual Volume 1 (Analyzing DNA) New York, Cold Spring Harbor Laboratory Press (1997). In addition, one skilled in the art understands that multiple alleles can be detected in individual reactions or in a single reaction (a "multiplex" assay). In view of the above, one skilled in the art realizes that the methods of the present invention for diagnosing or predicting susceptibility to or protection against CD in an individual may be practiced using one or any combination of the well known assays described above or another art-recognized genetic assay.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

### EXAMPLES

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention.

### Example 1 - Granuloma

The inventors identified clinical, serologic and genetic factors associated with granuloma formation in Crohn's disease (CD). 371 patients with CD who underwent disease-related surgical resection by a single surgeon were included in the study. Surgical samples were examined specifically for the presence or not of granulomas. Patients' demographic and clinical characteristics were collected by chart review, and samples drawn for IBD related serology (ASCA, anti-I2, anti-OmpC, CBirl and ANCA) and genetic analyses. Genome-wide analyses were performed using Illumina technology. Standard statistical tests for association were used and genetic association was assessed both at the genome-wide level and against known IBD and Leprosy susceptibility loci.

34.7% of CD surgical samples were found to contain granulomas. Granulomas were not associated with CD disease behavior. High ASCA titre was associated with the presence of granulomas (p=0.02). Patients with granulomas were younger at time of surgery (29.9 vs. 37.6 years, p=5x10-7) and far less likely to have ever smoked (12 vs. 32%, p=7x10-5). 14 Single Nucleotide Polymorphisms (SNPs) were associated with granulomas at a level of nominal association at a genome-wide level (p< 0.00005). These include a SNP adjacent to TGFb3, which has been implicated in the pathogenesis of stricturing Crohn's disease, and FTO, which is regulated by oral intake and is associated with raised body mass index. The strongest association was with NPAS2 (p =1x10-6), a core circadian gene that has been shown to modulate transcription of CX3CL1, a chemokine involved in CD pathogenesis. Amongst known IBD-associated loci, 7 were associated with granuloma formation (p<0.05), including: MUC1 (KL-6), also associated with granuloma-forming hypersensitivity pneumonitis; IL10, with known immunoregulatory function in the gut; and LRAP, associated with antigen presentation and LRRK2 a leucine-rich repeat kinase gene. One TNFSF15 SNP showed a trend towards association with the presence of granulomas (P = 0.066), of particular interest given a recent report that TNFSF15 is associated with Leprosy, another granulomatous condition. Of the known Leprosy loci (in addition to LRRK2 and TNFSF15), the inventors identified association with granulomatous CD and SNPs across the cytochrome P-450 cluster. Thus, the inventors have demonstrated putative genetic and demographic associations with the presence of granulomas in CD including a number of genes associated with Leprosy suggesting unique pathways in the pathogenesis of this subset of CD.

### Example 2 - Low Bone Density

The inventors identified 333 IBD subjects with bone density studies who had previously had genome wide association studies and IBD related serologies performed. Data on age, gender, ethnicity, disease distribution, surgeries, and smoking history were obtained from chart reviews. Osteoporosis, osteopenia, and normal bone mineral density (NBD) were defined by the WHO criteria based on DEXA scans. Standard tests for association between clinical characteristics, genetic markers and serologies were used. IBD related serology (ASCA, OmpC, 12, CBir-1, and ANCA) were obtained by ELISA and summarized into quartiles. Genetic data were generated using Illumina technology.

Of the 333 IBD study subjects, the inventors identified 252 cases of LBD and 81 cases of NBD. Disease location was not associated with LBD overall; however, perianal disease was associated with osteoporosis (P=0.021). Small bowel disease requiring surgery was associated with LBD (P=0.022), osteopenia (P=0.041) and osteoporosis (P=0.05). Smoking was not associated with bone density. Mean and median Anti-I2 titers were associated with LBD (P=0.023) and osteoporosis (P=0.006). On quartile analysis, anti-CBir-1 titers were associated with LBD (P=0.036) and osteoporosis (P=0.0006); further, ASCA was associated with osteoporosis (P=0.03). 38 genetic loci achieved nominal level of genome wide significance (P<5x10⁻⁵) including multiple single nucleotide polymorphisms (SNPs) at the HLA (P=1.37x10⁻⁷) as well as genes involved in cell adhesion (laminin, P=4.41x10⁻⁵) and innate immunity (plexin, P=9.02x10⁻⁷; NLR family, P=7.39x10⁻⁶). Stepwise linear regression was performed and all but 2 SNPs (rs11576349 and rs4954555) fell out of the model. These two SNPs were independently associated with LBD (2.41 x10⁻⁵ and 1.07 x 10⁻⁵) and together this 2 SNP model was highly associated with LBD (p-value linear regression 1.8 x 10⁻⁹) and explained 12.6 of the variance. Perianal disease is associated with osteoporosis; further, small bowel disease requiring surgery increases the risk for LBD. Anti-I2, anti-CBir-1, and ASCA are associated with increased risk for LBD and/or osteoporosis. Genes including HLA, laminin and plexin are associated with LBD. Thus, patients with these risk factors may benefit from more aggressive screening and treatment for osteoporosis.

While the description above refers to particular embodiments of the present invention, it should be readily apparent to people of ordinary skill in the art that a number of modifications may be made without departing from the spirit thereof. The presently disclosed embodiments are, therefore, to be considered in all respects as illustrative and not restrictive.

The various methods and techniques described above provide a number of ways to carry out the invention. Of course, it is to be understood that not necessarily all objectives or advantages described may be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as may be taught or suggested herein. A variety of advantageous and disadvantageous alternatives are mentioned herein. It is to be understood that some preferred embodiments specifically include one, another, or several advantageous features, while others specifically exclude one, another, or several disadvantageous features, while still others specifically mitigate a present disadvantageous feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be mixed and matched by one of ordinary skill in this art to perform methods in accordance with principles described herein. Among the various elements, features, and steps some will be specifically included and others specifically excluded in diverse embodiments.

Although the invention has been disclosed in the context of certain embodiments and examples, it will be understood by those skilled in the art that the embodiments of the invention extend beyond the specifically disclosed embodiments to other alternative embodiments and/or uses and modifications and equivalents thereof.

Many variations and alternative elements have been disclosed in embodiments of the present invention. Still further variations and alternate elements will be apparent to one of skill in the art. Among these variations, without limitation, are the selection of constituent modules for the inventive compositions, and the diseases and other clinical conditions that may be diagnosed, prognosed or treated therewith. Various embodiments of the invention can specifically include or exclude any of these variations or elements.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

In some embodiments, the terms "a" and "an" and "the" and similar references used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of this invention are described herein, including the best mode known to the inventors for carrying out the invention. Variations on those preferred embodiments will become apparent to those of ordinary skill in the art upon reading the foregoing description. It is contemplated that skilled artisans can employ such variations as appropriate, and the invention can be practiced otherwise than specifically described herein. Accordingly, many embodiments of this invention include all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification. Each of the above cited references and printed publications are herein individually incorporated by reference in their entirety.

In closing, it is to be understood that the embodiments of the invention disclosed herein are illustrative of the principles of the present invention. Other modifications that can be employed can be within the scope of the invention. Thus, by way of example, but not of limitation, alternative configurations of the present invention can be utilized in accordance with the teachings herein. Accordingly, embodiments of the present invention are not limited to that precisely as shown and described.

### EMBODIMENTS OF THE INVENTION

1. A method of diagnosing susceptibility to granuloma in an individual with Crohn's diseaes, comprising:
   (a) obtaining a sample from the individual;
   (b) assaying the sample to determine the presence or absence of at least one risk genetic variant;
   (c) assaying the sample to determine the presence or absence of at least one risk serological marker; and
   (d) diagnosing susceptibility to granuloma in the individual if the at least one risk genetic variant is present, or if the at least one risk serological marker is present, or if the at least one risk genetic variant is present and the at least one risk serological marker is present.
2. The method of according to embodiment 1, wherein the at least one risk genetic variant is at the genetic locus of TGFb3, FTO, NPAS2, MUC1, IL10, LRAP, LRRK2, TNFSF15, or cytochrome P-450 cluster, or a combination thereof.
3. The method of according to embodiment 1, wherein the at least one risk serological marker is selected from the group consisting of anti-Cbirl, ANCA, ASCA, anti-OmpC, and anti-I2.
4. The method of embodiment 3, wherein the ASCA is present in high titre.
5. The method of embodiment 1, wherein the at least one risk genetic variant comprises SEQ. ID. NO.: 1, SEQ. ID. NO.: 2, SEQ. ID. NO.: 3, SEQ. ID. NO.: 4, SEQ. ID. NO. : 5, and/or SEQ. ID. NO.: 6.
6. The method of embodiment 1, wherein the at least one risk genetic variant comprises SEQ. ID. NO.: 7, SEQ. ID. NO.: 8, SEQ. ID. NO.: 9, SEQ. ID. NO.: 10, SEQ. ID. NO. : 11, SEQ. ID. NO.: 12, and/or SEQ. ID. NO.: 13.
7. The method of embodiment 1, wherein the Crohn's disease is associated with a small bowel disease phenotype, an aggressive complicating phenotype, an internal penetrating disease phenotype, a stricturing disease phenotype, a fibrostenosing disease phenotype, or a combination thereof.
8. The method of embodiment 1, wherein the sample comprises a nucleic acid from the individual.
9. A method of diagnosing granuloma in an individual with Crohn's disease, comprising:
   (a) obtaining a sample from the individual;
   (b) assaying the sample to determine the presence or absence of at least one risk genetic variant;
   (c) assaying the sample to determine the presence or absence of at least one risk serological marker; and
   (d) diagnosing granuloma in the individual if the at least one risk genetic variant is present, or if the at least one risk serological marker is present, or if the at least one risk genetic variant is present and the at least one risk serological marker is present.
10. The method of embodiment 9, wherein the at least one risk genetic variant is at the genetic locus of TGFb3, FTO, NPAS2, MUC1, IL10, LRAP, LRRK2, TNFSF15, cytochrome P-450 cluster, or a combination thereof.
11. The method of embodiment 9, wherein the at least one risk serological marker is selected from the group consisting of anti-Cbirl, ANCA, ASCA, anti-OmpC, and anti-I2.
12. The method of embodiment 11, wherein the ASCA is present in high titre.
13. The method of embodiment 9, wherein the at least one risk genetic variant comprises SEQ. ID. NO.: 1, SEQ. ID. NO.: 2, SEQ. ID. NO.: 3, SEQ. ID. NO.: 4, SEQ. ID. NO. : 5, and/or SEQ. ID. NO.: 6.
14. The method of embodiment 9, wherein the at least one risk genetic variant comprises SEQ. ID. NO.: 7, SEQ. ID. NO.: 8, SEQ. ID. NO.: 9, SEQ. ID. NO.: 10, SEQ. ID. NO. : 11, SEQ. ID. NO.: 12, and/or SEQ. ID. NO.: 13.
15. The method of embodiment 9, wherein the Crohn's disease is associated with a small bowel disease phenotype, an aggressive complicating phenotype, an internal penetrating disease phenotype, a stricturing disease phenotype, a fibrostenosing disease phenotype, or a combination thereof.
16. A method of diagnosing susceptibility to low bone density (LBD) in an individual with inflammatory bowel disease (IBD), comprising:
   (a) obtaining a sample from the individual;
   (b) assaying the sample to determine the presence or absence of at least one risk genetic variant;
   (c) assaying the sample to determine the presence or absence of at least one risk serological marker; and
   (d) diagnosing susceptibility to LBD in the individual if the at least one risk genetic variant is present, or if the at least one risk serological marker is present, or if the at least one risk genetic variant is present and the at least one risk serological marker is present.
16. The method of embodiment 16, where the LBD is associated with osteoporosis and/or osteopenia.
17. The method of embodiment 16, wherein the at least one risk genetic variant is at the genetic locus of HLA, laminin, plexin, NLR family, or a combination thereof
18. The method of embodiment 16, wherein the at least one risk genetic variant is SEQ. ID. NO.: 14 and/or SEQ. ID. NO.: 15.
19. The method of embodiment 16, wherein the at least one risk serological marker is selected from the group consisting of anti-Cbirl, ASCA, and anti-I2.
20. The method of embodiment 16, wherein the IBD is associated with perianal disease.
21. A method of treating low bone density (LBD) in an individual with inflammatory bowel disease (IBD), comprising:
   (a) obtaining a sample from the individual;
   (b) assaying the sample to determine the presence or absence of at least one risk genetic variant;
   (c) assaying the sample to determine the presence or absence of at least one risk serological marker; and
   (d) treating LBD in the individual if the at least one risk genetic variant is present, or if the at least one risk serological marker is present, or if the at least one risk genetic variant is present and the at least one risk serological marker is present.
22. The method of embodiment 21, wherein the at least one risk genetic variant is SEQ. ID. NO.: 14 and/or SEQ. ID. NO.: 15.
23. The method of embodiment 21, wherein the at least one risk serological marker is selected from the group consisting of anti-Cbirl, ASCA, and anti-I2.

## Claims

1. A method of classifying a subgroup of Crohn's disease in a subject, the method comprising:
assaying a sample from a subject to detect the presence or absence of at least one risk genetic variant at a genetic locus comprising TGFb3, FTO, NPAS2, MUC1, IL10, LRAP, LRRK2, TNFSF15 or cytochrome p450 cluster, or any combination thereof; and
classifying the subgroup of Crohn's disease provided the at least one risk genetic variant is detected in the sample from the subject.

2. The method of claim 1, further comprising assaying a sample from the subject to detect an increase in expression of a serological marker, relative to a healthy individual.

3. The method of claim 2, wherein the serological marker comprises anti-Cbirl, ANCA, ASCA, anti-OmpC, or anti-I2, or any combination thereof.

4. The method of any of claims 1 and 2, further comprising the step of treating the subject with a treatment specific for the subgroup of Crohn's disease.

5. The method of any of claims 1-4, wherein the subgroup of Crohn's disease is associated with a small bowel disease phenotype, an aggressive complicating phenotype, an internal penetrating disease phenotype, a stricturing disease phenotype, or a fibrostenosing disease phenotype, or a combination thereof.

6. The method of any of claims 1-5, wherein the at least one risk genetic variant comprises SEQ.ID. NO.: 1, SEQ. ID. NO.: 2, SEQ. ID. NO.: 3, SEQ. ID. NO.: 4, SEQ. ID. NO.: 5, or SEQ. ID. NO.: 6.

7. The method of claim 6, wherein the at least one risk genetic variant comprises SEQ.ID. NO.: 1, SEQ. ID. NO.: 2, SEQ. ID. NO.: 3, SEQ. ID. NO.: 4, SEQ. ID. NO.: 5, and SEQ. ID. NO.: 6.

8. The method of any of claims 1-5, wherein the at least one risk genetic variant comprises SEQ.ID. NO.: 7, SEQ. ID. NO.: 8, SEQ. ID. NO.: 9, SEQ. ID. NO.: 10, SEQ. ID. NO.: 11, SEQ. ID. NO.: 12, or SEQ. ID. NO.: 13.

9. The method of claim 8, wherein the at least one risk genetic variant comprises SEQ.ID. NO.: 7, SEQ. ID. NO.: 8, SEQ. ID. NO.: 9, SEQ. ID. NO.: 10, SEQ. ID. NO.: 11, SEQ. ID. NO.: 12, and SEQ. ID. NO.: 13.

10. A diagnostic kit comprising a diagnostic panel comprising one or more risk genetic variants at a locus comprising TGFb3, FTO, NPAS2, MUC1, IL10, LRAP, LRRK2, TNFSF15 or cytochrome p450 cluster, or any combination thereof.

11. The diagnostic kit of claim 10, further comprising a diagnostic panel comprising at least one risk serological marker comprising ASCA, anti-Cbirl or anti-I2, or combination thereof.

12. The diagnostic kit of any of claims 10 and 11, wherein the at least one risk genetic variant comprises SEQ. ID. NO.: 1, SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, SEQ. ID. NO.: 8, SEQ. ID. NO.: 9, SEQ. ID. NO.: 10, SEQ. ID. NO.: 11, SEQ. ID. NO.: 12, or SEQ. ID. NO.: 13.

13. The diagnostic kit of claim 12, wherein the at least one risk genetic variant a comprises SEQ. ID. NO.: 1, SEQ. ID. NO.: 2, SEQ. ID. NO.: 3, SEQ. ID. NO.: 4, SEQ. ID. NO.: 5, SEQ. ID. NO.: 6, SEQ. ID. NO.: 7, SEQ. ID. NO.: 8, SEQ. ID. NO.: 9, SEQ. ID. NO.: 10, SEQ. ID. NO.: 11, SEQ. ID. NO.: 12, and SEQ. ID. NO.: 13.


SEQUENCE LISTING

<110>  CEDARS-SINAI MEDICAL CENTER
       ROTTER, Jerome I.
       TARGAN, Stephan R.

<120>  METHODS OF DIAGNOSING AND TREATING INTESTINAL GRANULOMAS AND LOW
       BONE DENSITY IN INFLAMMATORY BOWEL DISEASE

<130>  67789-312WO0

<150>  US 61/467,779
<151>  2011-03-25

<160>  15

<170>  PatentIn version 3.5

<210>  1
<211>  701
<212>  DNA
<213>  Homo sapiens

<400>  1
tctcctgtct ccaatgtggt ttttgttttg ttttgttttg ttttttgaga cagtcttgct      60

ctgtcgccca ggctggagtg cagtggcgta atctcagctc actgcaacct ctgcctcctg     120

ggttcaagcc attctcctgc ctcagcctcc tgagtagctg ggattacagg tgcacaacac     180

cacgcccagc taatttttgt atttttagta gagacggggt ttcaccatgt tggccaggat     240

ggtctcaatc tcttgacctc gtgatctgcc cgcctctgcc tcccaaagtg ctgggattac     300

aggcatgagc caccgcgccc ggccactgac gtgttgttaa aagagccgtg actgaagtcc     360

caccacccag ctttgcaagt gctataatct gacctcagtt ccaaatacct atttaaacgt     420

caggaacaaa gggctttttt ttattttttc aaaacatcag tctttaagct ttaaaaatgt     480

ttccatggag agtaactcag ygtcaagaga tgtgcagaaa atccaaagaa gggtaaggtt     540

gcccctggta tcaaggaaat aagaaccaga agtcatcagg acagtgaaga tatttagcaa     600

agaaatgtaa gtgggaaaac atgtaataaa gttagttttg tgatttaata aataaataaa     660

atgtttgcgt atgcatcgct tcccagaagc agccaggcat c                         701


<210>  2
<211>  623
<212>  DNA
<213>  Homo sapiens

<400>  2
ctggtctgca gttggaagga aggcaatgct atcacagaca gttcactcct aaaccacaag      60

ctactgcagc cttctcaagc atgagccaag ccccattaat ctgattcaaa acctacagag     120

attgcatatc aatttctcaa tgtaatccca ttggagtttt tcatgctaga cttgggatta     180

ggggcaattg ccccctttac acacactctc tcgtttcata ggttgaactc aacaagatag     240

ctcactgcaa agaagtcctc tccaaattct acttatcacg cccaataata gccacttttt 300

agatcgttaa aaagatctaa aaaaatcgtg tggcttgaga gtcattcaat cagtgaactg 360

acttacaatt cattttagaa ggtgtcatct agtgtctttg gcctcaattg aaagacagga 420

caagtcccat tttaatgtcc tgttatagga ataacctaat gaaatatttg tacatacaca 480

ggttgaggtt agatggcaac aagagtgttg taacatagga gctgcaatat ygacagcttg 540

cttaatccaa atagaaagtg attctgctaa tacaggcacc ttcaatttag ctcaccctta 600

gaaatgaatt gacattattc ttt 623

<210> 3
<211> 501
<212> DNA
<213> Homo sapiens

<400> 3
ttctgtattc ttagctaaaa tcccatctca aatcacatct tttgaaagaa gacttctgtg 60

agtatgatta ggggcttgaa ggtgccatct ataactcctc cctgtgaaga ttctggccaa 120

ttaagtttag gtaccaaaaa taaaaaaaaa aaacccacaa aaaaacaata aataggaatg 180

gaagcatcac tttatttgtt tgggaaagca catgtaacca tccagagctc tgataatggc 240

tggaatagca mgacgctgga gtggagaatt ctatttccta gcccagatct agatcttgaa 300

acttacattc ttaggtgtgc catgtgtgag aaagagcaaa aagaagagag gaatgaagag 360

agatggcctt caaagctgac tgagcctgct gcttagaagg tgacctcaag ccttcccaca 420

ttagaaataa tgaagataat ttctttcatc ctacactaat cattctcttc ttcaaacacc 480

attttaactt agaggtaaca t 501

<210> 4
<211> 601
<212> DNA
<213> Homo sapiens

<400> 4
aaagtacagg gagaatttgg agttccttcc aaactccggg tggaagaaaa ggtccttgta 60

caactctggg gtttcagcag cagaaattaa tgtcttctgt agtccaactc atgaactcaa 120

ctcagttctg gtaaaaatca gtccccaggc ctcacaataa ggtataggag agagactctg 180

actgatccag cctgggcaag atggatacct tgcctggatt gtccactgat ggaccaagac 240

acagagtcac atagtatggt cttgaccagc agagggttga acagagtagc aaggctagaa 300

mtggaaatgc tgacatcctt ttgagccagg cagtcagtga agagagggct gcagaaagaa 360

gttttacctg tagcccagtg ttgctaataa aatgttgaaa aactatctga gggcagccag 420

acctgttccc acaagtgata gctggttttc atttggggat cttcactttg tgacaagccg 480

gatttctatt tccatcagat aaaggctggc aataatgaac atcttcctga taactctcat 540

cacggttttc caaggagctg aaaatggtaa ttaatgcaaa agctgtgttt attggttcgt 600

g 601

<210> 5
<211> 1001
<212> DNA
<213> Homo sapiens

<400> 5
ggagtctccc tctgttgccc aagctggatt gcaatggcat gatctcggct cactgcaacc 60

tccacctcct gggttccacc catcctcctg cctcagcccc caagtagctg ggactacagg 120

tgtgcgccac cgcacctggc taatttttgt atttttagta gcgattgggt ttctccatgt 180

tggccaagct ggtttcaaac tcctgacttc agcctcccaa agtgctggga ttacatatgt 240

gagtcaccct gcccagcctg tcttggtttt ttgatcgtta aataatctcc ctctcctctt 300

gctttctctg ttttctcact ctgatataat tactgatgga tcttctaatt tgcttatctt 360

gtttgctgct actcctgtct cagtgtcctc ttctgctttg gggaagattt ttttcaacgt 420

cacccttcat atttagtttt acggttgtgc catctttgtt tcaatatcca ggaggctctt 480

tcttgttctc tgaatattaa yttttttatc accctcattc ttacatgcat cttgactctc 540

accttttctg aggatattaa ctaatagcaa gtagtttgtt ttcgacattt tcttctgttt 600

ccttcaagtt actcttattt gggctctgca tttcatctta gaggcttggc ctctgatgct 660

tagtgatccc tggctattca tatttagaca taaaacacta aatgtttctt gtaagttctc 720

agtgctggtg ggcttcaaaa tagggtgacc gtgagcgtta attttatgtg tcaacatgac 780

tggactacag gatacacaga catttgatca aacattgttc ttagtgtttt tggatgagtt 840

taatgtttaa atccataaac tgagtaaagc aaattgcccc ctctggaatg tgatgggcct 900

tatccaatct caggcctgaa tagaaaaaaa gaccgaccct cctgcaagta agagagaacg 960

cctcctgcct gagaaggggt tcagaacagg gacattgttt t 1001

<210> 6
<211> 1948
<212> DNA
<213> Homo sapiens

<400> 6
ttctcaaact ctatgggggc agattttatg tactgaagtg tttcatgatc tactgtgtgt 60

tggactgtta atttgttctt tttctttttc taaaattaat ttcccttctc ccttccttcc 120

ttccttcttt ccttccttcc ttccctcctt cccttccttc cttccttccc ttccttcctt 180

tcctccttca ttcctttcct ttcctttctt ccttccttct tcccttcttt cctttccttt 240

ttctcttctc ttctcttctc ttctctcttt gagatggagt ctcgcattgt tgcttaggct 300

ggtctaagct ctggcctcaa gcagtcctcc tgccttggcc tcccaaagtg ctgggattac 360

agacaggagc cactgtacct ggcttaactt gttttttcaa tgagactttg ggacaacata 420

ctgagtttgg gggttgtgtg agtcagacag attagcacta tgctgtaatc ataaataaca 480

cctaaatctc acctgtttaa atcagtaaag agttattttt tcttttgctg caatttcatc 540

cccgctttgg caggggaatc tttttcacgt ctcatgcaga tatggaggct gacagggacc 600

tcactctctg ttactggtct ccatggcagg aagaaggaag ggaatgtgat aaattgtatg 660

tggaattttt gtttgtttca ttttgctttg ctattattca actttcttga ggtataattg 720

aagtatcata aactgcatat atttaaaatg taccttttga tgcattttga gatatgcatg 780

tacctttgat atcctcacca tgatgaagag aataaacatt ttcataaccc ccaaaaggtt 840

ccttgtttca catgcctctc tccactcctg tcttcatgca accagggacc tgctatctgt 900

caatgtaggt tgatctgcat gttccggaat tttttttttt tttttaataa acggaagcac 960

acagcactta ctcttcttct actggcttct ttcatgcagc tcaatgattt gaggaatcac 1020

tcatgttgta tgtgcctcaa cagttcattc tgttttattg ctgagtagta ttccattgta 1080

tggctatacc acaatttgtt tatctattcc tgcacatgga aagttggttt gtttctggtt 1140

trtaatgatc acaaatcaag ctgataagaa catttgggta caagtctttg tgtagacttg 1200

tgcttttatt tatcttggat gcatacttag gagtgaagat gtcccaaagt ggttgtacca 1260

tttcacattc ccttagcagt ttataagagt tccagttgct ccacatcttc accaacacat 1320

ggtatgatca gttgaaaaac ttttcccagc actttgggag gccgaggtgg gtggatcaca 1380

aggtcaggag atcgagacca tcctggctaa cacagtgaaa ccctgtctct attaaaaata 1440

caaaaaatta gctgggcatg gtggcaggtg cctatagtcc cagctacttg ggaggctgag 1500

gcaggagaat ggcgtgaacc cgggaggcgg agcttgcagt gagctgagat tgcgccactg 1560

cactccagcc tggaagacag agcgaggctc catctcgaaa caacaaaaaa cacaacaaaa 1620

aactgttcac ccttcaaata ggtggatagt ggtatctcag tgtagtttta atttgcattt 1680

tcctaatgat taattatatt gagcattttt aatgtgttta ttggctgtat tgtggagtat 1740

ccttgggtgc tgtgctccgg ggtttgctct ggtgtgtcaa cttggtagaa tgacttttat 1800

ccaatttttt tccagtgagg cttctagtta gtacaatatc tacacactgt agcactagtt 1860

agcatccccg ttcctacttt gtttttatct gaaccagttt agcccttcca tttctctcct 1920

gacctgtggc tagtttatgg gctcccta 1948

<210> 7
<211> 1125
<212> DNA
<213> Homo sapiens

<400> 7

cctaagagag aaaggtgtca ttgaaagaat tccgtctgta aaattatttt ggggtttctt 60

aaattcacat agtcttgtac atagtttaat gtacatggcc ttggcattgt gtaaaatgtt 120

atttggacac tctggaagaa gaggatagca aatttgattc tagggttata agactggagg 180

gttaattata atataaatca rtcaggtaat tgggagaaga ttggaggaaa tttgatacat 240

gtttatgtgt ttatcaagtg attgcatctg cttttcactc tctgctgcca ccatctggcc 300

ccaaaacctt tgtatgagcc acaatagtca catccctggg acatctttgt gccccctcca 360

gtctcttttc cacacggcag ccaatatcat ttcttatgat tctctactgg ttcatgtcat 420

tcctacactt ggcatcactc attagttgga ctttggataa acagaaagca tcaagctctt 480

gcctcctttc cagcttcatc tcatgtcttc cctcactcgt aacgctctgc ttatgccagc 540

cttctctctg gcccttgact atatggagtt ctttccagcc tcagggtatt tgcaacctgc 600

tgttctgcta cacaggacat ctgccattca cactttctat ctctcactcc tgctcatccc 660

ttagatctca gctaaacata actttcttgg atggcactcc tttaaagccc agccacccga 720

ccccagaatt taaattggcc tctccccatc cttaatctaa tttccaacag ggcatactga 780

ccttttcctt tgtataactt attataattt gtggtgtcat gttttctata tgcttgttta 840

acctctgtct tccccaatag acagttaact ttataaagcc atggacggtg tccatctttt 900

tttttttttt aagtgctgta ctctgcctca gacaagatgt ggttggtgct taattaatag 960

ttgttgaatc aatgattaca tcaacaaacg aacacttagt atgtgacaag cactgggcta 1020

agtgcataat actcatgatt ttatttaatc ctcacaataa ccttttgagt taattatcat 1080

taatgtcatt cacaaatgag gaaataatac ttcttcatgc agtga 1125

<210> 8
<211> 605
<212> DNA
<213> Homo sapiens

<400> 8
ttgattatgt ttttccaggt tcactttctt ccttgggatg tcacagaact gaactctatc 60

agaaatatgc tagggcatgt tttattctct tctgcatcct ctgtcagaaa cagccaacaa 120

gtgccatttt caagtcgcta cagatctcac ctagaccctt ggaaatatca ggttctgctg 180

gattctgatc aagagtgccc acgcagtaac ttagaatctg accagcatgc caggactcac 240

atctagctct tcccaaaggc acacgtgggc agggattctc aaatcatctg gggatgtcga 300

taaaccacag atgytgactc agtgagtgtg gggtgaggac tgacactctt aatttccaaa 360

agctcccagg tgccactgtg ggtctttggg actgcatttt gaatggaaag gtgctagggt 420

atttttaatg acatcctaac gaaattatgc cattagggat ttaactgtgt attccacagc 480

tttactttcc ttttcaattt aacctcttta gttcaacaat gtaacacttg tggaactcca 540

caaattagga tgcaaaatga acaagaaaag ggatttagtt ttaactgaga tgtcccttca 600

caaga 605

<210> 9
<211> 701
<212> DNA
<213> Homo sapiens

<400> 9
tctcctgtct ccaatgtggt ttttgttttg ttttgttttg ttttttgaga cagtcttgct 60

ctgtcgccca ggctggagtg cagtggcgta atctcagctc actgcaacct ctgcctcctg 120

ggttcaagcc attctcctgc ctcagcctcc tgagtagctg ggattacagg tgcacaacac 180

cacgcccagc taatttttgt atttttagta gagacggggt ttcaccatgt tggccaggat 240

ggtctcaatc tcttgacctc gtgatctgcc cgcctctgcc tcccaaagtg ctgggattac 300

aggcatgagc caccgcgccc ggccactgac gtgttgttaa aagagccgtg actgaagtcc 360

caccacccag ctttgcaagt gctataatct gacctcagtt ccaaatacct atttaaacgt 420

caggaacaaa gggctttttt ttattttttc aaaacatcag tctttaagct ttaaaaatgt 480

ttccatggag agtaactcag ygtcaagaga tgtgcagaaa atccaaagaa gggtaaggtt 540

gcccctggta tcaaggaaat aagaaccaga agtcatcagg acagtgaaga tatttagcaa 600

agaaatgtaa gtgggaaaac atgtaataaa gttagttttg tgatttaata aataaataaa 660

atgtttgcgt atgcatcgct tcccagaagc agccaggcat c 701

<210> 10
<211> 1948
<212> DNA
<213> Homo sapiens

<400> 10
ttctcaaact ctatgggggc agattttatg tactgaagtg tttcatgatc tactgtgtgt 60

tggactgtta atttgttctt tttctttttc taaaattaat ttcccttctc ccttccttcc 120

ttccttcttt ccttccttcc ttccctcctt cccttccttc cttccttccc ttccttcctt 180

tcctccttca ttcctttcct ttcctttctt ccttccttct tcccttcttt cctttccttt 240

ttctcttctc ttctcttctc ttctctcttt gagatggagt ctcgcattgt tgcttaggct 300

ggtctaagct ctggcctcaa gcagtcctcc tgccttggcc tcccaaagtg ctgggattac 360

agacaggagc cactgtacct ggcttaactt gttttttcaa tgagactttg ggacaacata 420

ctgagtttgg gggttgtgtg agtcagacag attagcacta tgctgtaatc ataaataaca 480

cctaaatctc acctgtttaa atcagtaaag agttattttt tcttttgctg caatttcatc 540

cccgctttgg caggggaatc tttttcacgt ctcatgcaga tatggaggct gacagggacc 600

tcactctctg ttactggtct ccatggcagg aagaaggaag ggaatgtgat aaattgtatg 660

tggaattttt gtttgtttca ttttgctttg ctattattca actttcttga ggtataattg 720

aagtatcata aactgcatat atttaaaatg taccttttga tgcattttga gatatgcatg 780

tacctttgat atcctcacca tgatgaagag aataaacatt ttcataaccc ccaaaaggtt 840

ccttgtttca catgcctctc tccactcctg tcttcatgca accagggacc tgctatctgt 900

caatgtaggt tgatctgcat gttccggaat tttttttttt tttttaataa acggaagcac 960

acagcactta ctcttcttct actggcttct ttcatgcagc tcaatgattt gaggaatcac 1020

tcatgttgta tgtgcctcaa cagttcattc tgttttattg ctgagtagta ttccattgta 1080

tggctatacc acaatttgtt tatctattcc tgcacatgga aagttggttt gtttctggtt 1140

trtaatgatc acaaatcaag ctgataagaa catttgggta caagtctttg tgtagacttg 1200

tgcttttatt tatcttggat gcatacttag gagtgaagat gtcccaaagt ggttgtacca 1260

tttcacattc ccttagcagt ttataagagt tccagttgct ccacatcttc accaacacat 1320

ggtatgatca gttgaaaaac ttttcccagc actttgggag gccgaggtgg gtggatcaca 1380

aggtcaggag atcgagacca tcctggctaa cacagtgaaa ccctgtctct attaaaaata 1440

caaaaaatta gctgggcatg gtggcaggtg cctatagtcc cagctacttg ggaggctgag 1500

gcaggagaat ggcgtgaacc cgggaggcgg agcttgcagt gagctgagat tgcgccactg 1560

cactccagcc tggaagacag agcgaggctc catctcgaaa caacaaaaaa cacaacaaaa 1620

aactgttcac ccttcaaata ggtggatagt ggtatctcag tgtagtttta atttgcattt 1680

tcctaatgat taattatatt gagcattttt aatgtgttta ttggctgtat tgtggagtat 1740

ccttgggtgc tgtgctccgg ggtttgctct ggtgtgtcaa cttggtagaa tgacttttat 1800

ccaatttttt tccagtgagg cttctagtta gtacaatatc tacacactgt agcactagtt 1860

agcatccccg ttcctacttt gtttttatct gaaccagttt agcccttcca tttctctcct 1920

gacctgtggc tagtttatgg gctcccta 1948

<210> 11
<211> 622
<212> DNA
<213> Homo sapiens

<400> 11
aagattgtaa ggcagagaaa agaatgctga gccatgagaa tggtaagagg ctgatcaatg 60

gtgttgtggg acatgctaag aggtcttcac ttcgctatat tggggatagg aagcagcaat 120

gtgctaagac ctacctgtca gcaaatatta ctgccatggt cccaaggcct taggcatgat 180

agagctcaat aaatgctggg tgaatgaaca aataaatgaa tgcataagcc atgaaataat 240

tggataaaat tctctaggtg cataaggaga atgaattaaa gcagtcaagg ctagaagtaa 300

gaagaccatt taggaggtta ttgcatataa gaaatgctaa ggtatgaact ayagcagtgt 360

ctgtaggact agaaaggagg gaacaaattc aggagcaatt cagaaaacag aattgataga 420

gtcacagtcc tcacatggcc accagcttat cttcctgaaa gaacattgtt tattaggcca 480

tttggctgaa caaaaacgtt cagcagtttc ccacaccttg tagaatatac atactgtaaa 540

gtttgctacc tacattcacg gtccttcatc agcttctagc tctacatctg ccccagaata 600

actattcatc aaatgctgaa ta 622

<210> 12
<211> 914
<212> DNA
<213> Homo sapiens

<400> 12
ttgaaacaaa gaaagtagag agaatattat agtgaactcc cacaaatcta gaaacaagct 60

ttaatcattg tgaacatgct ttcaatcttt tctaatcacc ccacttgttt ttattgtatt 120

tacttattta tatattttaa ttaatttttg ctgcaggatt ttaaagcaaa ttccagatat 180

cataccacct ctccactgaa accagtatgt tgtcctaaat gaaaatcata ttaaaatttt 240

acataacaat atggaattaa cctaacaatt ttgaccttaa tcacttagta ttatttgaat 300

agtatttaat atctgcttca tatctgaaag tttccaattg aatcaaacaa aatattcttc 360

ttataccata tttgtttaat tcagcattta aataagcaca agtattaatt ttggttgtta 420

tgtcttctac atctatttta ctctccattt tttttcttct ttaatcatca aaaccagccc 480

taaagaatgt ccaactttcc rcatttagtt tattgcttct tccttatatg agttaacttg 540

ttcctctatt tcttccagat gttgcaaaag tagttagacc tagaaaatta attagctaac 600

agttcaatta cgtctgtaca tgataaattt tagaggccat gcctcacact ctccatttca 660

tcacattttg aggcacttat gcatctgtgg tctcttttgg tgatgctgat atcactgggt 720

aacgtgcagt ctgaactttt ctcataacat tccccatcaa ctcttcacct aatgcttaca 780

tcatccactg atccatcgaa tgttgtctaa attcattatt acattacaga tcgcaataca 840

gtatttttct aattttatca tcccttatgg agtcttttcc aaaaattaga aagttaacct 900

aaaagaaaac tcag 914

<210> 13
<211> 582
<212> DNA
<213> Homo sapiens

<400> 13
ggcagaggag gtccttcgaa gctctctggg agcacaagcc tctgagcagg gatctgggtg 60

aggggagcaa ggggctctgt gaagacccag ggaccagatt ccaggcagaa gccctcagag 120

cagaagtggg attgaagggc tccaagagca gaataaagac cagtgtagcc agactgggga 180

gaggggaggc cccatctcac atgggttgct tctgaatgtg atgtggggca gtggcctgct 240

atgatgktct tttaaaaagc actagaactg ttgaatgaaa tcctgatgcc aggagggcca 300

gggtggaatt ggggagccca ctgaggctgt ggcaggagtc tgggaagagt ctgagagggg 360

tgatgaggca gggaggtggc agatttggga gaaacgtagg aggcagagat acaaggacca 420

gcagaaggga acagaggagc aaggagagac ctgcttacag ctgagccatc ggggagagct 480

ggtgccttct gctgaagtag gatgaacatg tcttgggggg aaatcaagac ttctgctttt 540

taaagttaag tgatcagggg tgggtggcag agtctcgacc ta 582

<210> 14
<211> 1001
<212> DNA
<213> Homo sapiens

<400> 14
ctcctatgcc ctgatgggga actctctaga cagacaaatg aatagctgag gcttctcatc 60

acccatatac ccacattctc tctctctttt tttttttttt tttaaacaga gtctccttct 120

gttgcccagg ctggagtaca gcctgggctc actggatcct ctgcctcctg ggttcaagtg 180

attctcctgc ctcagtctct caagtaggtg ggactacagg tgcatgccac caggcccgac 240

taatttttgt atttttagta gagacggggt ttcaccatga tggccaggtt ggcctcgaac 300

tcctgacctc aagtgatcct cccaccttag cctcccaaag tgctaggatt acaggcgtga 360

gccaccatgc ccggctgcac actctcttac ctttcccaca ccttgaaatt tccctcactg 420

cccaccctaa atacagtacc atgagacgca aatcttagat tagatttcaa aggaagggca 480

gtgtcactta gttccactaa rgcctacctg tatcctctgt ggcctagact ggaggtggat 540

atggggagac tgatttccaa gatagacata gctctgttat cttcactccc tgagtccaag 600

cccagctgaa tacctaagtg gcttctcatt cttttttttt tttttttgga gacggagtct 660

tgccctgtcg cccaggctgg agtgcagtgt catgatcttg gctcactgca acctctgcct 720

cccaggttca agcaattctc ctgcctcagc ctcttgagta gctgggatta caggtatgtg 780

ccaccccacc tggctaattt ttgtattttt agtagagaca gggtttcacc atgttggccg 840

ggatggtctc gatctcctga cctcgtgatc cacctgtctc ggcctcccaa agtgctggga 900

ttacagatgt gagccaccac gcccagccgt ggcttctcat tcttatcctt agagattgct 960

ggcatccccg gcctcttacc actcttccca tccttccatc a 1001

<210> 15
<211> 753
<212> DNA
<213> Homo sapiens

<400> 15
atacatgaga tttctcatat atatatattt atatgacatc atgagattat agatctaata 60

tataatttta ttatataatg ttatatatta tatttattat atataataat atatatttat 120

aatatataat ctcatgatgt catgtatatc tgataatcac tatgtgatat atgtgctcac 180

acatacaaac acattttttt atgttgttat atgtatttca tattataata tgtatgtatt 240

cacaaaattt acccaaaagt gtattttccc tagttccata tctttataac ttgggtgcat 300

tctgatgtac tctagtctat acttttttgt atttcatttc aaaaaatgct tgttataact 360

gaatgcattg attttatatg ctataatggg tctacccata gactgaaaaa tttggctcta 420

gttgaagttt ctcaaaaatc agtgtgtatt agaaatactt gggaagcttt ttacaaagct 480

aggttcctga ggcccctccc yctaagattc tgacttgttg catcatagaa aaggcccagg 540

aacctacatt ttttggctca atatatggaa aataaaaaca acagtgctca gtagacggtg 600

ctaaggtcca tatgagaagt tcacacagtg tgttaaatcc atgttcagag tgaagagtgt 660

ttgctgtcct gaacagagtc tgcactcctc caactgcacc tctcaatggg tgattatttc 720

aaagatgtca agaaagaaaa gccaactcat aaa 753